# EUROPEAN PATENT SPECIFICATION

(11) **EP 0 280 557 B1**
(45) Date of publication and mention of the grant of the patent: **08.09.1993**
(21) Application number: 88301651.1
(22) Date of filing: 26.02.1988
(51) Int. Cl.: G01N 33/569, G01N 33/546

(54) **Test kit, extraction device and method for the determination of streptococcus a antigen**
Testsatz, Extraktionsvorrichtung und Verfahren zur Bestimmung von Streptococcus-A-Antigen
Trousse de réactifs d'essai, dispositif d'extraction et méthode pour la détermination d'antigène A de streptocoques

(30) Priority: 27.02.1987 US 19850; 18.09.1987 US 98431; 11.12.1987 US 131618
(43) Date of publication of application: 31.08.1988
(73) Proprietor: EASTMAN KODAK COMPANY (a New Jersey corporation), Rochester, New York 14650 (US)
(72) Inventor: Snyder, Brian A., c/o EASTMAN KODAK COMPANY, Rochester, NY 14650 (US); Contestable, Paul B., c/o EASTMAN KODAK COMPANY, Rochester, NY 14650 (US); Belly, Robert T., c/o EASTMAN KODAK COMPANY, Rochester, NY 14650 (US)
(74) Representative: Nunney, Ronald Frederick Adolphe

(56) References cited:
- EP-A- 0 150 567
- EP-A- 0 231 750
- WO-A-87/01393

## Description

This invention relates to a test kit for the determination of Streptococcus A antigen, an extraction device for extracting antigen from the Streptococcus organisms and to a method of determining the antigen.

The antigen-antibody reaction is the basis for all immunological test methods. Certain proteins known as antibodies are produced by mammals in response to the presence of an antigen, that is a foreign substance, which can be another protein or a carbohydrate. This normal body response to a foreign substance has led to the development of a number of techniques which are used to diagnose various diseases, disorders and physiological conditions. In a general sense, the component of the antibody-antigen reaction to be detected is defined herein as the immune species while the other corresponding component is considered the receptor.

For example, in vitro tests for the presence of a suspected protein, antigen or antibody in a biological sample are carried out by adding the immunological counterpart to the biological sample. If the suspected substance is present, the resulting antigen-antibody reaction can be demonstrated by precipitation of the antigen-antibody complex. This reaction complex is generally difficult to detect visually. For this reason, either antibodies or antigens are often bound to insoluble particles, for example polymer latex particles, so that when the complex is formed, it is readily detectable from the resulting agglutination either by observing the presence of clumping or a detectable tracer associated with the particles. Agglutination then is characterized by the clumping of particles from a suspension of particles.

Of the several groups of Streptococci, group A Streptococcus (S. pyogenes) is primarily responsible for causing pathological conditions in humans, such as B-hemolytic pneumonia, scarlet fever, rheumatic fever, cardiac sequelae, glomerulonephritis, septic sore throat and puerperal sepsis. Because of the serious nature of infections potentially caused by Streptococcus A, it is important to diagnose its presence in an early stage of infection so that an appropriate course of treatment may be selected. Early tests for detection required culturing a biological sample for long periods of time, usually at least 18 and up to 48 hours. In most cases, such lengthy tests delay treatment making them undesirable. Some doctors must forward test cultures to laboratories for evaluation by mail or courier, thereby delaying treatment further.

More recent tests for Streptococcus A have been described which are allegedly quicker than the culturing techniques. One known agglutination test utilizes certain enzymes to extract the antigen directly from the swab used to obtain a specimen from the throat. A kit for the assay comprises an applicator means for collecting the specimen, an extraction reagent containing the enzymes and suitable indicator reagents.

Another agglutination assay is described in E.P. Publication 150,567. This assay describes an extraction technique which uses a solution of sodium nitrate combined with glacial acetic acid. This assay has a serious disadvantage, however, because the extraction composition is not easily stored, transported or handled. Acetic acid is a volatile liquid at room temperature, the normal conditions of use. It therefore presents problems in long-term storage and ease of handling.

U.S. Patent 4,673,639 allegedly describes a means for overcoming the problems associated with volatile extraction reagents. This reference proposes affixing one or both extraction reagents, in combination with a water-soluble binder material, in a ready-to-use microtube. The reagents used include a nonvolatile acid, such as citric acid. The patent also teaches a further advantage of this method over known assays in that a neutralizing step is unnecessary. Eliminating this step allegedly has no adverse effects on the agglutination reagents used or the assay results.

EP-A-0 231 750 describes an assay for Streptococcal organisms using a coated polymeric acid as one of the extraction reagents. The use of polymeric acid, however, creates more difficulty in manufacture, and increases costs over the readily available nonpolymeric acids which can be used.

It has been found that elimination of a neutralizing step adversely affects certain agglutination reagents in an assay for Streptococcus A antigen. It can also adversely affect antibodies if they are in a low pH environment too long. Furthermore, it would be desirable to eliminate the need for using a binder material to immobilize extraction reagents because of the additional material and manufacturing expense they engender.

The problems noted above with known test kits and Streptococcus A assays are overcome with a test kit for the determination of Streptococcus A antigen comprising:
(a) a water-insoluble substrate having thereon a dried coating of a first extraction reagent which is necessary for nitrous acid extraction of the antigen from a Streptococcus A organism,
(b) an aqueous solution of a second extraction reagent which is necessary for the nitrous acid extraction, and
(c) a sample of an immunoreactive reagent comprising water-insoluble particles having either Streptococcus A antigen or antibodies to the antigen attached thereto,
the kit chararterized wherein the first extraction reagent coating is binder-free and the first reagent is a nonpolymeric, nonvolatile organic acid which has a pKa equal to or less than 5 and a melting point equal to or greater than 18°C, and the kit further comprises: (d) a neutralizing solution having a pH of from 5 to 10.

Also provided by this invention is an extraction device for extracting Streptococcus A antigen from a biological specimen suspected of containing Streptococcus A organisms comprising:
(i) a water-insoluble container having affixed internally a dried coating of an extraction reagent which is necessary for nitrous acid extraction of the antigen from a Streptococcus A organism, and
(ii) an applicator means for collecting and depositing the biological specimen within the container,
the device characterized wherein the extraction reagent coating is binder-free and the extraction reagent is a nonpolymeric, nonvolatile organic acid which has a pKa equal to or less than 5 and a melting point equal to or greater than 18°C.

A method for the determination of Streptococcus A antigen comprises:
A. contacting a biological specimen suspected of containing Streptococcus A organisms with first and second extraction reagents necessary for nitrous acid extraction of antigen from the organisms, the first reagent provided in a dried, binder-free coating on a water-insoluble substrate, the first reagent being a nonpolymeric, nonvolatile organic acid which has a pKa equal to or less than 5 and a melting point equal to or greater than 18°C, and the second reagent provided in an aqueous solution,
B. neutralizing the resulting solution of extracted antigen,
C. reacting the extracted antigen with antibodies to the antigen so as to form a reaction product of antigen and antibodies,
D. separating the reaction product from unreacted materials, and
E. determining the amount of either the reaction product or the unreacted materials.

A preferred method for the determination of Streptococcus A antigen and a biological specimen comprises:
A. contacting a biological specimen suspected of containing Streptococcus A organisms with a water-insoluble substrate having thereon a dried, binder-free coating of a first extraction reagent which is necessary for nitrous acid extraction of antigen from the organisms the extraction reagent being a nonpolymeric, nonvolatile organic acid which has a pKa equal to or less than 5 and a melting point equal to or greater than 18°C,
B. substantially simultaneously with contacting step A, contacting the specimen with an aqueous solution of a second extraction reagent which is necessary for the nitrous acid extraction,
C. incubating the resulting extraction solution for up to 5 minutes at a temperature of up to 90°C,
D. neutralizing the extraction solution,
E. in a test device, contacting the neutralized extraction solution with an immunoreactive reagent for the antigen comprising water-insoluble particles having antibodies to the antigen attached thereto to form a agglutinated reaction product of the antibodies and the antigen,
F. separating the agglutinated reaction product from unreacted materials, and
G. determining the amount of either the agglutinated reaction product or the unreacted materials.

The present invention provides a number of advantages over known assays for Streptococcus A antigen. It avoids the use of acetic acid or other objectionable reagents. Moreover, while it utilizes a nonvolatile acid, it further avoids the use of a binder material to immobilize the reagent on a substrate of some type. As noted above, the use of a binder material is disadvantageous because of the additional material and manufacturing costs and operations required. A binder material may be suitable for a laboratory assay, but high volume commercial manufacturing operations must be less costly in a highly competitive market.

Further, the present invention utilizes a neutralization step in the assay in order to reduce or eliminate the adverse effects of highly acidic conditions on antibody molecules and agglutination reagents which have free carboxyl groups. The highly acidic environment of the antigen extraction procedure severely effects free carboxyl groups on antibody molecules as well as those on polymeric particles. Hence, the problems noted with the assay described in U.S. Patent 4,673,639 are avoided.

The present invention provides a diagnostic test for Streptococcus A which can be performed in a very short time, that is usually less than 10 minutes, and without the use of complicated equipment. This permits the test to be performed in a doctor's office and enables the doctor to determine a course of treatment based upon the results of the test the same day. The test detects the presence of Streptococcus A antigen in a biological sample, such as a swab specimen from the throat, urine specimen or sample of another aqueous liquid. Such biological samples can be tested with or without pretreatment (for example, filtration) to remove unwanted debris or interferents.

The test kit of the present invention includes a water-insoluble substrate having thereon a dried, binder-free coating of a first extraction reagent (described below). The substrate can be of any suitable configuration, construction or material as long as it is water-insoluble and inert to the reactions which are used to assay for antigen. In the simplest form, the substrate can be a test tube, glass slide, microtube, test slide, filter paper strip or any other suitable material to which the extraction composition can be affixed to one or more surfaces or cavities and dried without the use of a binder material. The substrate composition can be any suitable natural or synthetic material (for example, glass, polymeric materials, cellulosic materials and others known in the art).

In a preferred embodiment, the substrate is a container which is part of an extraction device, the container being designed so that the antigen can be extracted from a biological specimen within the container using the needed reagents. Such an extraction device can also include an applicator means for collecting and depositing the biological specimen within the container. An applicator means usually includes an applicator stick and a fibrous swab at one end thereof. Useful applicator means for Streptococcus A tests are known in the art, for example, in U.S. Patent 4,618,576. In its simplest form, the extraction device can be a simple cup with a holder for holding an applicator means. A first extraction reagent is deposited on the inside of the cup.

It is known that nitrous acid is a useful chemical for extracting Streptococcus A antigen from the organisms, but it is also known to be unstable. Hence, it is preferable to use a first and second reagent which react with each other to provide nitrous acid immediately during the extraction procedure. Usually, these reagents include a nitrite and an acid which reacts with the nitrite to provide nitrous acid.

One of these reagents is present in dried form on the substrate or extraction device described above. It is not important which one it is, although it is preferably the nonvolatile acid (described below). The first reagent is not mixed with any binder materials as taught in U.S. Patent 4,673,639. The dried reagent is nonvolatile and compositionally stable at ambient conditions (that is, 18-30°C). It is optional for the first reagent to be mixed with one or more inert addenda in dried form (for example, surfactants, buffers or other materials known to one skilled in the art).

The extraction reagent is applied to the substrate in a suitable way and drying it under suitable conditions.

The first reagent is preferably a nonvolatile organic acid which has a melting point equal to or greater than 18°C, and a pKa of 5 or less. Examples of useful organic acids, include, but are not limited to: citric acid, malonic acid, phenylacetic acid, oxalic acid, glycolic acid, chloroacetic acid, trichloroacetic acid, fluoroacetic acid, bromoacetic acid, iodoacetic acid, succinic acid, glutaric acid, adipic acid, pimelic acid, suberic acid, benzoic acid, benzene sulfonic acid, p-toluene sulfonic acid, azelaic acid and sebacic acid. The carboxylic acids noted above are particularly useful, with citric acid most preferred.

A second extraction reagent needed for the extraction procedure is a nitrite. Useful nitrites include, but are not limited to, inorganic nitrites such as sodium, potassium, lithium, calcium, strontium, barium and silver nitrites, and organic nitrites such as butyl and isoamyl nitrites. Sodium and potassium nitrites are preferred, and sodium nitrite is most preferred. While one extraction reagent (for example, the organic acid) is usually supplied in the kit in dried form on the substrate described above, the other reagent (for example, the nitrite) is generally supplied in an aqueous solution. This solution can optionally contain surfactants or other addenda inert to the reaction to produce nitrous acid.

The concentration of nitrite and organic acid combined in the resulting extraction solution can be varied widely depending upon the particular compounds used, their water solubility and the suspected amount of antigen present in the test sample. More particularly, the amount of nitrite present is at least 0.05 molar, and can be from 2 to 12 molar. The organic acid is generally present in an amount of at least 0.01 molar, and preferably in an amount of from 0.01 to 1 molar.

The test kit also contains a neutralizing solution having a pH of from 5 to 10, and preferably from 6.5 to 8.5. This solution is used to neutralize the extraction solution after extraction has occurred and prior to contact with agglutination reagents. More details of the assay procedure are given below. Generally, the neutralizing solution is a suitable buffer having the desired pH. Many such buffers are known in the art. One preferred buffer is 3-(N-morpholino)propanesulfonic acid. The neutralizing solution can optionally include metal chelating agents, such as ethylenediaminetetraacetic acid.

The immunoreactive reagent supplied and used in the practice of the present invention includes either Streptococcus A antigen or antibodies to the antigen bound to the surface of suitable water-insoluble particles (defined below). Either the particles, antigen or antibodies can be labeled in a suitable manner with a tracer material (defined below) to render the reagent detectable. The reagent can be supplied as a dried sample, or as an aqueous dispersion.

In one embodiment, the immunoreactive reagent comprises antibodies bound to the surface of water-insoluble particles. A competitive binding assay for extracted Streptococcus A antigen in a biological specimen is carried out in this invention using the immunoreactive reagent in combination with a predetermined amount of labeled Streptococcus A antigen. Reaction products of the insoluble immunoreactive reagent with labeled and unlabeled antigen are then formed and separated from unreacted materials in a suitable manner. Either the insolubilized or soluble labeled antigen can be measured to determine the amount of antigen in the specimen.

Alternatively, antibodies can be competitively reacted with extracted antigen and an immunoreactive reagent having antigen bound to particles which are labeled in some manner.

In another embodiment, extracted antigen is allowed to bind to a suitable solid carrier material. The immunoreactive reagent is the corresponding antibody to the antigen. This antibody can be suitably labeled and the reaction product or unreacted antibody can be measured.

In still another embodiment, the Streptococcus A antigen can be determined using an immunoreactive reagent comprising antibodies attached to carrier particles in an assay wherein a labeled antibody to the antibody of the immunoreactive reagent is added. Such antibodies can be labeled with any suitable tracer, such as a radioisotope, chemiluminescent compound, colorimetric or fluorometric compound and others known in the art. For example, the antigen can be determined in a "sandwich" enzyme-label immunosorbent assay, sometimes known in the art as a "sandwich" ELISA. This technique involves the use of a first antibody (monoclonal or polyclonal) attached to the particles, and a second antibody (monoclonal or polyclonal) which is labeled with a suitable enzyme. Both antibodies are specific to the Streptococcus A antigen. The immunoreactive reagent can be reacted with the extracted antigen prior to, simultaneously with or subsequent to reaction of the antigen with the enzyme-labeled antibody.

In a preferred embodiment, the presence of Streptococcus A antigen is detected by an agglutination method using a reagent comprising water-insoluble particles having antibodies to Streptococcus A antigen bound in a suitable manner to the surface of the particles. Reaction (or binding) between antigen and antibody molecules then results in a linking together of the particles so that they form large agglutinates.

Suitable water-insoluble particles useful in the reagent can be natural or synthetic particles which are water-insoluble and capable of having a suitable number of antibody molecules bound thereto in some manner. Examples of useful particles include ferritin crystals, agarose particles, glass beads, polymeric particles, such as latex particles, and others known in the art. The following references describe representative useful particles: U.S. Patents 3,700,609, 3,853,987, 4,108,972, 4,258,001, 4,401,765, 4,419,453, 4,459,361, 4,478,946 and 4,591,571. The particles useful in this invention are generally quite small, that is less than 2 micrometers in diameter. Preferably, they have an average diameter of from 0.1 to 1 micrometer.

Particularly useful particles are polymeric latex particles, and more preferably they are what are known in the art as core-shell polymeric latex particles. A wide variety of monomers can be used in the preparation of such particles as long as the particles are water-insoluble. A worker skilled in the polymer chemistry art would be able to design and prepare suitable latex particles. Preferred core-shell polymeric latex particles in the practice of this invention are described in the example below. These particles have a core composed of homo- or copolymers of styrene, and a shell composed of homo- or copolymers of chloromethylstyrene or m & p-(2-chloroethylsulfonylmethyl)styrene. The present invention is particularly advantageous with polymeric particles which have free carboxyl groups on the outer surface. Such groups are sometimes useful for dispersing the particles in aqueous media and keeping them in suspension. The groups may be present in homo- and copolymers prepared from ethylenically unsaturated polymerizable monomers having carboxyl groups, including but not limited to, acrylic acid, methacrylic acid, itaconic acid and others known in the art.

While the assay of this invention can be carried out, for example, by observing the presence or absence of agglutinate using light scattering or other suitable techniques, it is preferred that the particles have sufficient tracer molecules associated therewith in order to allow quantitative determination of the species from the amount of tracer seen in either the agglutinate or in the unagglutinated residual materials. The tracer molecules can be suitably attached to the outer surface of the particles, or preferably, distributed within the particles (such as a dye). Any tracer material which allows detection of the agglutinate can be used. If ferritin crystals are used as the particles, the tracer molecules are molecules of iron inherently in those crystals. Other natural or synthetic particles can have, as tracers: radioisotopes, colorimetric compounds, fluorometric compounds, chemiluminescent compounds, phosphorescent compounds and other detectable materials known in the art. Preferably, the tracer is a radioisotope, colorimetric compound or fluorometric compound (for example, dye or rare earth chelate). A worker skilled in the art would be able to combine an appropriate tracer with the particular particle used.

In one embodiment, the tracer can be a fluorescent rare earth chelate such as europium chelate, as described for example, in U.S. Patent 4,259,313. In another and preferred embodiment, the tracer is a colorimetric compound which is readily detected in the agglutinate. Useful dyes are known in the art. Some dyes can be incorporated into the particles when the particles are prepared. Alternatively, the dyes are imbibed into preformed particles in such a manner that they do not leach out.

The tracer can be distributed within the particles in any suitable manner. For example, the tracer can be uniformly distributed therein as shown for example in U.S. Patent 3,853,987. Preferably, the tracer molecules are located in a restricted area of the particles, for example, near the surface or predominantly in the interior thereof. In the preferred core-shell particles, the tracer can be in either the core or shell, but most preferably, substantially all of the tracer is in the core of the particles.

Streptococcus A antigen or antibodies thereto are bound to the outer surfaces of the particles in a suitable manner, for example by adsorption or covalent attachment. Attachment can be achieved using known techniques, as described for example in the references cited above. Covalent attachment is preferred as the molecules are less likely to be removed from the particles. When covalently attached, the antigen or antibody molecules can be bound directly to the particles or through suitable linking groups. Either monoclonal or polyclonal antibodies can be used. Antibodies can be obtained commercially or prepared using known techniques. Polyclonals, for example, are generally prepared by injecting antigen into suitable mammals which then generate the antibodies which can be removed for use. Monoclonals are obtained using standard hybridoma technology.

The test kit of this invention can optionally comprise a wash solution suitable for washing unreacted materials from reacted materials. A preferred wash solution has a pH of from 5 to 10 and which contains an ionic compound providing an ionic strength of at least 0.25.

While the present invention is not so limited, the assay for Streptococcus A antigen can be carried out using a suitable test device which comprises a microporous membrane for separating reacted materials from unreacted materials, as in an agglutination assay. Such a device can have one or more wells into which extracted antigen is deposited for reaction with the immunoreactive reagent. This reagent can be added to the device separately during the assay or with the extracted antigen, or incorporated therein at the time of manufacture.

In general, extraction of antigen is accomplished by contacting the biological specimen, for example, on a swab, with the extraction reagents in a solution (for example, in the extraction device) in a manner such that the reagents accomplish the desired result. Often, an incubation period (up to 5 minutes) at a temperature up to 90°C is required. Longer periods at lower temperature can also be used. Preferably, the incubation is for up to 1 minute at up to 90°C, or up to 5 minutes at up to 30°C. Most preferably, the incubation is carried out for 1 minute at 25°C (room temperature). The advantage of the preferred embodiment of the present invention is that extraction can occur quickly making the overall assay very rapid.

After neutralization of the resulting extraction solution with the neutralizing solution described above, the extracted antigen is contacted with the immunreactive reagent in a test device as described above so as to form a reaction product of antigen and antibodies.

In the preferred embodiment described above, the product is an agglutinate. Simultaneously or subsequent to contact of extracted antigen with antibody molecules to form the agglutinate, the agglutinate is also preferably contacted with a microporous water-insoluble membrane. In one embodiment, the agglutinate can be formed in a separate container and then brought into contact with the membrane. Alternatively and preferably, the agglutinate is formed in the presence of the membrane. This membrane (described in detail below) can be simply a filter means held by hand through which unagglutinated materials are filtered. Preferably, however, it is mounted in a test device in which the assay is carried out. Such a test device is described above.

Any microporous water-insoluble membrane can be used as long as it is inert to the materials used in the assay, and has the desired porosity which will allow fluids and nonagglutinated material to pass through but which will retain agglutinated materials. In other words, the membrane pores must be large enough to allow passage of the unagglutinated particles, but not large enough to allow agglutinated particles to pass through. More particularly, the average pore size of the membrane must be at least five times the average diameter of the water-insoluble particles described above. Preferably, the average pore size is from 6 to 15 times the average particle diameter. Useful membranes include polymeric materials which are commercially available from various sources. One useful membrane is a nylon-66 microporous membrane manufactured and marketed by Pall Corp. as BIODYNE or ULTIPOR.

A suitable incubation period can be used to optimize agglutination, if desired, before or during contact with the membrane. After that period, unagglutinated residual materials are washed through the membrane while leaving the agglutinate thereon. This separation step is usually carried out within 1 to 10 minutes of adding the neutralized solution to the test device.

Once the unagglutinated residual materials have been washed through the membrane, the amount of antigen in either the agglutinate or residual materials can generally be determined with the unaided eye if the tracer is a readily viewable colorimetric dye. Otherwise, standard colorimetric detection equipment can be used. Other types of tracers, for example, radioisotopes, fluorescent dyes, phosphorescent dyes, and the like, require suitable detection equipment.

### Example 1: Agglutination Determination of Streptococcus A Using Citric Acid in Extraction Procedure

This example demonstrates the practice of the present invention for the determination of Streptococcus A antigen.

Core-shell polymeric latex particles were prepared using core-shell polymerization techniques. A red dye (Oil Red EGN) was imbibed into the core of the particles. The core of the particles was composed of poly(styrene-co-2-acetoacetoxyethyl methacrylate) (70:30 weight ratio) while the shell was composed of poly(m, p-chloromethylstyrene). The average diameter of the particles was about 0.45 micrometer. Monoclonal antibodies to Streptococcus A antigen and casein were immobilized on these particles as follows: to 0.6 ml of 50 mM borate buffer (pH 8.5) was added 0.1 mg of total protein comprised of a 10:1 mixture of anti-Strep A antibody (2.9 mg/ml solution in phosphate buffered saline solution, known in the art as PBS) and casein (10 mg/ml water). After mixing, 41.5 µl of a 5% suspension of the polymeric latex particles were added (to provide 0.3% solids) and the resulting solution was rotated (end-over-end) for 24 hours at 37°C to effect covalent attachment of the antibody to the outer surfaces of the particles and the formation of an agglutination indicator reagent.

A solution of succinic anhydride (10 mg/ml dimethyl sulfoxide) was added to a suspension of the agglutination indicator reagent described above at a weight ratio of 1 part anhydride to 1 part total protein. The resulting suspension was mixed for four hours at 25°C, centrifuged for 5 minutes at 7000 rpm and the resulting pellet was resuspended in 0.1 molar glycine buffer (pH 8.5) to a concentration of 0.3% solids.

An isolate of Streptococcus A obtained from a local hospital was used in the assays of this example. Streptococcus A antigen was extracted from an isolate at 25°C for 1 minute in an extraction device using a solution of equal volumes of sodium nitrite (8 molar) and citric acid (0.2 molar). The citric acid had been coated and dried in the device without a binder prior to adding the isolate. The extraction solution was then neutralized with an equal volume of 3-(N-morpholino)propanesulfonic acid buffer (2 molar, pH 7.5) containing ethylenediaminetetraacetic acid (75 mM).

A nylon 66 microporous membrane (5 µm average pore size) was incorporated into a test well of a disposable test device, and pretreated by washing with 100 µl of a 2% succinylated casein solution.

A mixture of sodium chloride (80 µl, 1 molar), the agglutination indicator reagent suspension described above (40 µl), and extracted antigen (80 µl) containing about 4.2 x 10⁵ colony-forming units was added to the test well of the test device containing the membrane, and incubated therein for two minutes at 25°C. The fluid was then allowed to drain into a compartment below the membrane, and the agglutinate on the membrane was washed with 150 µl of a wash fluid having an ionic strength of about 1.0.

After the washing step, the amount of dye in the agglutinate on the membrane was measured at 540 nm using reflectance measuring equipment. The Williams-Clapper transform (J. Optical Soc. Am., 43, p. 595, 1953) was used to calculate transmission density values. The agglutinate on the membrane was readily observable and had a significantly greater density value than the density of a background control (the different was 0.148). These data indicate that the present invention is useful for the extraction and determination of Streptococcus A antigen in a biological sample.

### Example 2: Determination of Streptococcus A Using Succinic Acid for Antigen Extraction

The procedure and reagents described in Example 1 were used in this example except that citric acid was replaced with succinic acid.

Streptococcus A antigen was extracted from the microbial isolate for one minute at 25°C using a solution of 90 µl citric acid (0.2 molar) and 90 µl of sodium nitrite (8 molar). The extraction solution was then neutralized with tricine buffer (1 molar, pH 8.6) and ethylenediaminetetraacetic acid (25 mmolar).

A mixture of the agglutination reagent (90 µl) and the extraction solution (40 µl) was mixed for two minutes at 25°C. It was then added to a disposable test device containing a microporous membrane (BIODYNE A filter which had been pretreated with succinylated casein (1.07 mg/cm²), and incubated thereon for two minutes at 25°C. The fluid was then allowed to drain through the membrane, and the agglutinate on the membrane was washed with 150 µl of a wash fluid having an ionic strength of about 1.0.

The amount of dye in the agglutinate was then measured as described in Example 1 and similar results were obtained as with citric acid. This indicates that succinic acid can be effectively used in extracting the Streptococcus A antigen in the practice of this invention.

### Example 3: Comparative Test

This example shows the effect of omitting a neutralization step in this invention.

Streptococcus A antigen was extracted from a microbial isolate for about 1 minute at 25°C in an extraction device using a solution of citric acid (10 µl, 1.2 molar) and sodium nitrite (120 ml, 8 molar) to form a first test solution. The citric acid had been coated and dried in the device without a binder prior to adding the isolate. A second extraction solution was similarly prepared except that distilled water was used in place of the microbial isolate (that is, it had no antigen). Neither extraction solution was neutralized.

A solution of sodium chloride (80 µl, 1 molar), agglutination reagent as described in Example 1 (40 µl) and the first extraction solution described above (40 µl) were successively added to the test wells of a test device like that described in Example 1, and incubated therein for about 2 minutes at 25°C.

Likewise, the sodium chloride solution, agglutination reagent and second extraction solution were successively added to the test wells of a second test device and incubated.

After incubation, the fluids were allowed to drain through the membranes in the test devices, and the agglutination reagent remaining on the membranes was washed with sodium chloride solution (80 µl, 1 molar), then visually examined. An intense red color was observed in all wells of both test devices. This indicates that non-specific agglutination occurred with the agglutination reagent as a result of not neutralizing the extraction solutions, so that agglutination occurred with or without the presence of antigen.

The tests described above were repeated except that the extraction solutions were mixed with the agglutination reagent in separate test tubes without neutralization. The presence of agglutination was observed in both test tubes.

## Claims

1. A test kit for the determination of Streptococcus A antigen comprising:
(a) a water-insoluble substrate having thereon a dried coating of a first extraction reagent which is necessary for nitrous acid extraction of the antigen from a Streptococcus A organism,
(b) an aqueous solution of a second extraction reagent which is necessary for the nitrous acid extraction, and
(c) a sample of an immunoreactive reagent comprising water-insoluble particles having either Streptococcus A antigen or antibodies to the antigen attached thereto,
the kit characterized wherein the first extraction reagent coating is binder-free and the first extraction reagent is a nonpolymeric, nonvolatile organic acid which has a pKa equal to or less than 5 and a melting point equal to or greater than 18°C, and the kit further comprises: (d) a neutralizing solution having a pH of from 5 to 10.

2. The kit as claimed in claim 1 wherein the nonvolatile organic acid is citric acid, malonic acid, phenylacetic acid, glycolic acid, chloroacetic acid, trichloroacetic acid, fluoroacetic acid, bromoacetic acid, iodoacetic acid, succinic acid, glutaric acid, adipic acid, pimelic acid, suberic acid, benzoic acid, benzenesulfonic acid, p-toluene sulfonic acid, azelaic acid or sebacic acid.

3. The kit as claimed in either of claims 1 or 2 wherein the second extraction reagent is a nitrite.

4. A test kit for the determination of Streptococcus A antigen comprising:
(a) a water-insoluble substrate having thereon a dried coating of a nonpolymeric nonvolatile organic acid which has a pKa equal to or less than 5 and a melting point equal to or greater than 18°C,
wherein the acid is citric acid, malonic acid, phenylacetic acid, glycolic acid, chloroacetic acid, trichloroacetic acid, fluoroacetic acid, bromoacetic acid, iodoacetic acid, succinic acid, glutaric acid, adipic acid, pimelic acid, suberic acid, benzoic acid, benzenesulfonic acid, p-toluene sulfonic acid, azelaic acid or sebacic acid,
(b) an aqueous solution of sodium nitrite or potassium nitrite,
(c) a sample of an immunoreactive reagent for the antigen comprising water-insoluble polymeric particles having antibodies to the antigen covalently attached thereto, and further having free carboxyl groups on the outer surfaces and dye molecules therewithin,
(d) a disposable test device for carrying out an assay for the antigen, the test device comprising a microporous membrane,
(e) a wash solution, and
(f) an applicator means comprising an applicator stick having a swab at one end for collecting a test specimen,
the kit characterized wherein the organic acid coating is binder-free, and the kit further comprises: (g) a neutralizing solution having a pH of from 5 to 10.

5. An extraction device for extracting Streptococcus A antigen from a biological specimen suspected of containing Streptococcus A organisms comprising:
(i) a water-insoluble container having affixed internally a dried coating of an extraction reagent which is necessary for nitrous acid extraction of the antigen from a Streptococcus A organism, and
(ii) an applicator means for collecting and depositing the biological specimen within the container,
the device characterized wherein the extraction reagent coating is binder-free and the extraction reagent is a nonpolymeric, nonvolatile organic acid which has a pKa equal to or less than 5 and a melting point equal to or greater than 18°C.

6. A method for the determination of Streptococcus A antigen in a biological specimen comprising:
A. contacting a biological specimen suspected of containing Streptococcus A organisms with a water-insoluble substrate having thereon a dried, binder-free coating of a first extraction reagent which is necessary for nitrous acid extraction of antigen from the organisms, and the extraction reagent is a nonpolymeric, nonvolatile organic acid which has a pKa equal to or less than 5 and a melting point equal to or greater than 18°C,
B. substantially simultaneously with contacting step A, contacting the specimen with an aqueous solution of a second extraction reagent which is necessary for nitrous acid extraction,
C. incubating the resulting extraction solution for up to 5 minutes at a temperature up to 90°C,
D. neutralizing the resulting extraction solution for up to 5 minutes at a temperature up to 90°C,
E. in a test device, contacting the neutralized extraction solution with an immunoreactive reagent for the antigen comprising water-insoluble particles having antibodies to the antigen attached thereto to form a agglutinated reaction product of the antibodies and the antigen,
F. separating the agglutinated reaction product from unreacted materials, and
G. determining the amount of either the agglutinated reaction product or the unreacted materials.

7. The method as claimed in claim 6 wherein the biological specimen and the first and second extraction reagents are contacted within an extraction device comprising the water-insoluble substrate to which is affixed the first extraction reagent by inserting an applicator means carrying the specimen into the container containing the first and second extracting reagents.

8. The method as claimed in either of claims 6 or 7 wherein the extraction solution is neutralized to a pH of from 5 to 10.

9. A method for the determination of Streptococcus A antigen comprising:
A. contacting a biological specimen suspected of containing Streptococcus A organisms with first and second extraction reagents necessary for nitrous acid extraction of antigen from the organisms, the first reagent provided in a dried, binder-free coating on a water-insoluble substrate, the first reagent being a nonpolymeric, nonvolatile organic acid which has a pKa equal to or less than 5 and a melting point equal to or greater than 18°C, and the second reagent provided in an aqueous solution,
B. neutralizing the resulting solution of extracted antigen,
C. reacting the extracted antigen with antibodies to the antigen so as to form a reaction product of antigen and antibodies,
D. separating the reaction product from unreacted materials, and
E. determining the amount of either the reaction product or the unreacted materials.

## Patentansprüche

1. Testsatz für die Bestimmung von Streptococcus A Antigen mit:
(a) einem wasser-unlöslichen Substrat, auf dem sich eine getrocknete Beschichtung eines ersten Extraktions-Reagens befindet, das für die Salpetrigsäure-Extraktion des Antigens aus einem Streptococcus A Organismus erforderlich ist,
(b) einer wäßrigen Lösung eines zweiten Extraktions-Reagens, das für die Salpetrigsäure-Extraktion erforderlich ist, und
(c) einer Probe eines immunoreaktiven Reagens mit in Wasser unlöslichen Teilchen, an die entweder Streptococcus A Antigen oder Antikörper zum Antigen gebunden sind,
dadurch gekennzeichnet, daß die erste Extraktions-Reagens-Beschichtung bindemittelfrei ist und daß das erste Extraktions-Reagens eine nicht polymere, nicht flüchtige organische Säure ist, die einen pKa-Wert hat, der gleich ist oder kleiner als 5 sowie einen Schmelzpunkt, der gleich ist oder größer als 18°C und daß der Testsatz weiterhin aufweist:
(d) eine neutralisierende Lösung mit einem pH-Wert von 5 bis 10.

2. Satz nach Anspruch 1, in dem die nichtflüchtige organische Säure Zitronensäure, Malonsäure, Phenylessigsäure, Glykolsäure, Chloressigsäure, Trichloressigsäure, Fluoressigsäure, Bromessigsäure, Jodessigsäure, Bernsteinsäure, Glutarsäure, Adipinsäure, Pimelinsäure, Suberinsäure, Benzoesäure, Benzolsulfonsäure, p-Toluolsulfonsäure, Azelainsäure oder Sebacinsäure ist.

3. Satz nach Anspruch 1 oder 2, in dem das zweite Extraktions-Reagens ein Nitrit ist.

4. Testsatz für die Bestimmung von Streptococcus A Antigen mit:
(a) einem wasser-unlöslichen Substrat, auf dem sich eine getrocknete Beschichtung aus einer nichtpolymeren nichtflüchtigen organischen Säure befindet, die einen pKa-Wert von gleich oder kleiner als 5 aufweist und einen Schmelzpunkt von gleich oder größer als 18°C,
wobei die Säure besteht aus Zitronensäure, Malonsäure, Phenylessigsäure, Glykolsäure, Chloressigsäure, Trichloressigsäure, Fluoessigsäure, Bromessigsäure, Jodessigsäure, Bernsteinsäure, Glutarsäure, Adipinsäure, Pimelinsäure, Suberinsäure, Benzoesäure, Benzolsulfonsäure, p-Toluolsulfonsäure, Azelainsäure oder Sebacinsäure,
(b) einer wäßrigen Lösung von Natriumnitrit oder Kaliumnitrit,
(c) einer Probe eines immunoreaktiven Reagens für das Antigen mit in Wasser unlöslichen polymeren Teilchen, an die Antikörper des Antigens kovalent gebunden sind, und die weiterhin freie Carboxylgruppen an der äußeren Oberfläche aufweisen sowie Farbstoffmoleküle darauf oder darin,
(d) einer Wegwerf-Test-Einheit zur Durchführung einer Antigenbestimmung, wobei die Test-Einheit eine mikroporöse Membran aufweist,
(e) einer Waschlösung, und
(f) einem Applikator mit einem Applikator-Stift mit einem Schwabbel an einem Ende zur Annahme einer Testprobe,
dadurch gekennzeichnet, daß die Beschichtung aus der organischen Säure bindemittelfrei ist und daß der Testsatz weiterhin aufweist:
(g) eine neutralisierende Lösung mit einem pH-Wert von 5 bis 10.

5. Extraktionseinheit für die Extraktion von Streptococcus A Antigen aus einer biologischen Probe, von der erwartet wird, daß sie Streptococcus A Organismen enthält, mit:
(i) einem wasser-unlöslichen Behälter, in dem im Inneren eine getrocknete Beschichtung aus einem Extraktions-Reagens befestigt ist, das für die Salpetrigsäure-Extraktion des Antigens aus Streptococcus A Organismen erforderlich ist und
(ii) einem Applikator zur Annahme und Abscheidung der biologischen Probe innerhalb des Behälters,
dadurch gekennzeichnet, daß die Extraktions-Reagens-Beschichtung bindemittelfrei ist und daß das Extraktions-Reagens eine nichtpolymere, nichtflüchtige organische Säure mit einem pka-Wert von gleich oder kleiner als 5 und einem Schmelzpunkt von gleich oder größer als 18°C ist.

6. Verfahren zur Bestimmung von Streptococcus A Antigen in einer biologischen Probe, umfassend:
A. Inkontaktbringen einer biologischen Probe, von der angenommen wird, daß sie Streptococcus A Organismen enthält mit einem wasser-unlöslichen Substrat, auf dem sich eine getrocknete, bindemittelfreie Beschichtung aus einem ersten Extraktions-Reagens befindet, das für die Salpetrigsäure-Extraktion des Antigens aus dem Organismus erforderlich ist, wobei das Extraktions-Reagens eine nichtpolymere, nichtflüchtige organische Säure ist mit einem pKa-Wert von gleich oder kleiner als 5 sowie einem Schmelzpunkt von gleich oder größer als 18°C,
B. praktisch gleichzeitig mit der Kontaktstufe A kontaktieren der Probe mit einer wäßrigen Lösung eines zweiten Extraktions-Reagens, das für die Salpetrigsäure-Extraktion erforderlich ist,
C. Inkubieren der erhaltenen Extraktions-Lösung 5 Minuten lang bei einer Temperatur bis zu 90°C,
D. Neutralisieren der erhaltenen Extraktions-Lösung bis zu 5 Minuten lang bei einer Temperatur bis zu 90°C,
E. Kontaktieren der neutralisierten Extraktions-Lösung in einer Test-Einheit mit einem immunoreaktiven Reagens für das Antigen mit wasser-unlöslichen Teilchen mit Antikörpern zum Antigen, die an die Partikel gebunden sind, zum Zwecke der Bildung eines agglutinierten Reaktionsproduktes aus Antikörpern und dem Antigen,
F. Trennung des agglutinierten Reaktionsproduktes von nicht umgesetzten Materialien und
G. Bestimmung der Menge von entweder dem agglutinierten Reaktionsprodukt oder den nicht umgesetzten Materialien.

7. Verfahren nach Anspruch 6, bei dem die biologische Probe und das erste und das zweite Extraktions-Reagens innerhalb einer Extraktions-Einheit miteinander in Kontakt gebracht werden mit dem in Wasser unlöslichen Substrat, an das das erste Extraktions-Reagens gebunden ist, durch Einführung eines Applikators mit der Probe in den Behälter, der das erste wie auch das zweite Extraktions-Reagens enthält.

8. Verfahren nach einem der Ansprüche 6 oder 7, bei dem die Extraktions-Lösung auf einen pH-Wert von 5 bis 10 neutralisiert wird.

9. Verfahren zur Bestimmung von Streptococcus A Antigen mit:
A. Kontaktieren einer biologischen Probe, von der angenommen wird, daß sie Streptococcus A Organismen enthält, mit einem ersten und einem zweiten Extraktions-Reagens, die erforderlich sind für die Salpetrigsäure-Extraktion von Antigen aus den Organismen, wobei das erste Reagens in einer getrockneten bindemittelfreien Beschichtung auf einem wasserunlöslichen Substrat vorliegt, wobei das erste Reagens eine nichtpolymere, nichtflüchtige organische Säure ist, die einen pKa-Wert von gleich oder kleiner als 5 aufweist sowie einen Schmelzpunkt von gleich oder größer als µ8°C und wobei das zweite Reagens in einer wäßrigen Lösung zur Anwendung gebracht wird,
B. Neutralisieren der erhaltenen Lösung des extrahierten Antigens,
C. Umsetzen des extrahierten Antigens mit Antikörpern für das Antigen, unter Erzeugung eines Reaktionsproduktes aus Antigen und Antikörpern,
D. Trennung des Reaktionsproduktes von nicht umgesetzten Materialien,
E. Bestimmung der Menge von entweder dem Reaktionsprodukt oder den nicht umgesetzten Materialien.

## Revendications

1. Nécessaire de test pour le dosage de l'antigène du Streptocoque A comprenant :
(a) un substrat insoluble dans l'eau recouvert d'une couche sèche d'un premier réactif d'extraction qui permet d'extraire l'acide nitreux de l'antigène contenu dans un organisme Streptocoque A,
(b) une solution aqueuse d'un second réactif d'extraction de l'acide nitreux, et
(c) un échantillon d'un agent immunoréactif comprenant des particules insolubles dans l'eau liées soit à l'antigène du Streptocoque A soit aux anticorps de l'antigène,
le nécessaire étant caractérisé en ce que la couche du premier réactif d'extraction ne contient pas de liant et le premier réactif d'extraction est un acide organique non volatile qui n'est pas sous forme de polymère et qui a un pKa inférieur ou égal à 5 et un point de fusion supérieur ou égal à 18°C, le nécessaire comprenant de plus (d) une solution neutralisante ayant un pH compris entre 5 et 10.

2. Nécessaire selon la revendication 1 dans lequel l'acide organique non volatile est l'acide citrique, l'acide malonique, l'acide phénylacétique, l'acide glycolique, l'acide chloroacétique, l'acide trichloroacétique, l'acide fluoroacétique, l'acide bromoacétique, l'acide iodoacétique, l'acide succinique, l'acide glutarique, l'acide adipique, l'acide pimélique, l'acide subérique, l'acide benzoïque, l'acide benzènesulfonique, l'acide p-toluènesulfonique, l'acide azélaique ou l'acide sébacique.

3. Nécessaire selon l'une quelconque des revendications 1 ou 2 dans lequel le second réactif d'extraction est un nitrite.

4. Nécessaire de test pour le dosage de l'antigène du Streptocoque A comprenant :
(a) un substrat insoluble dans l'eau recouvert d'une couche sèche d'un acide organique non volatile qui n'est pas sous forme de polymère et qui a un pKa inférieur ou égal à 5 et un point de fusion supérieur ou égal à 18°C dans lequel l'acide est l'acide citrique, l'acide malonique, l'acide phénylacétique, l'acide glycolique, l'acide chloroacétique, l'acide trichloroacétique, l'acide fluoroacétique, l'acide bromoacétique, l'acide iodoacétique, l'acide succinique, l'acide glutarique, l'acide adipique, l'acide pimélique, l'acide subérique, l'acide benzoïque, l'acide benzènesulfonique, l'acide p-toluènesulfonique, l'acide azélaique ou l'acide sébacique,
(b) un solution aqueuse de nitrite de sodium ou de nitrite de potassium,
(c) un échantillon d'un agent immunoréactif de l'antigène comprenant des particules polymères insolubles dans l'eau liées de façon covalente avec des anticorps de l'antigène et de plus, ne contenant pas des groupes hydroxy sur la surface externe et, comprenant des molécules de colorant,
(d) un dispositif de test à usage unique pour le dosage de l'antigène, le dispositif de test comprenant une membrane microporeuse,
(e) une solution de lavage, et
(f) un moyen d'application comprenant un stick applicateur ayant un tampon à l'une de ces extrémités pour collecter l'échantillon à tester,
le nécessaire étant caractérisé en ce que la couche d'acide organique ne contient pas de liant, et le nécessaire comprend de plus, (g) une solution neutralisante ayant un pH compris entre 5 et 10.

5. Dispositif pour l'extraction de l'antigène du Streptocoque A à partir d'un échantillon biologique pouvant contenir des organismes Streptocoque A comprenant:
(i) un conteneur insoluble dans l'eau sur lequel est fixée de façon interne une couche sèche d'un réactif d'extraction qui permet d'extraire l'acide nitreux de l'antigène contenu dans un organisme Streptocoque A, et
(ii) un moyen d'application pour collecter et déposer l'échantillon biologique dans le conteneur,
le dispositif étant caractérisé en ce que la couche de réactif d'extraction ne contient pas de liant et le réactif d'extraction est un acide organique non volatile, qui n'est pas sous forme de polymère et qui a un pKa inférieur ou égal à 5 et un point de fusion supérieur ou égal à 18°C.

6. Procédé pour le dosage de l'antigène du Streptocoque A dans un échantillon biologique qui consiste à :
A. mettre en contact un échantillon biologique pouvant contenir des organismes Streptocoque A avec un substrat insoluble dans l'eau qui est recouvert d'une couche sèche ne contenant pas de liant d'un premier réactif d'extraction qui permet d'extraire l'acide nitreux de l'antigène contenu dans les organismes, le réactif d'extraction étant un acide organique non volatile qui n'est pas sous forme de polymère et qui a un pKa inférieur ou égal à 5 et un point de fusion supérieur ou égal à 18°C,
B. simultanément à l'étape A, mettre en contact l'échantillon avec une solution aqueuse d'un second réactif d'extraction qui permet d'extraire l'acide nitreux,
C. incuber la solution d'extraction résultante au maximum 5 minutes à une température inférieure ou égale à 90°C,
D. neutraliser la solution d'extraction résultante au maximum 5 minutes à une température inférieure ou égale à 90°C,
E. dans le dispositif de test, mettre en contact la solution d'extraction neutralisée avec l'agent immunoréactif de l'antigène comprenant des particules insolubles dans l'eau liées à des anticorps de l'antigène afin de former un agglutinat qui est le produit de réaction des anticorps et de l'antigène,
F. séparer l'agglutinat des composés qui n'ont pas réagi, et
G. déterminer la quantité soit d'agglutinat, soit de composés qui n'ont pas réagi.

7. Procédé selon la revendication 6 dans lequel l'échantillon biologique et le premier et le second réactifs d'extraction sont mis en contact dans le dispositif d'extraction qui comprend un substrat insoluble dans l'eau sur lequel est fixé le premier réactif d'extraction, au moyen d'un applicateur qui permet de mettre l'échantillon biologique dans le conteneur qui contient le premier et le second réactifs d'extraction.

8. Procédé selon l'une quelconque des revendications 6 ou 7 dans lequel la solution d'extraction est neutralisée à un pH compris entre 5 et 10.

9. Procédé pour le dosage d'un antigène du Streptocoque A qui consiste à :
A. mettre en contact un échantillon biologique pouvant contenir des organismes Streptocoque A avec un premier et un second réactifs d'extraction qui permettent d'extraire l'acide nitreux de l'antigène contenu dans les organismes, le premier réactif formant une couche sèche et sans liant sur un substrat insoluble dans l'eau, le premier réactif étant un acide organique non volatile qui n'est pas sous forme de polymère et qui a un pKa inférieur ou égal à 5 et un point de fusion supérieur ou égal à 18°C, et le second réactif étant sous forme de solution aqueuse,
B. neutraliser la solution résultante d'antigène extrait,
C. faire réagir l'antigène extrait avec des anticorps de l'antigène afin de former un produit de réaction de l'antigène et des anticorps,
D. séparer le produit de réaction des composés qui n'ont pas réagi, et
E. déterminer la quantité soit du produit de réaction soit des composés qui n'ont pas réagi.
